(19) Europäisches Patentamt
European Patent Office
Office européen des brevets

(11) **EP 3 146 958 A1**

(12) **EUROPEAN PATENT APPLICATION**

(43) Date of publication:
**29.03.2017 Bulletin 2017/13**

(51) Int Cl.:
***A61K 8/73*** *(2006.01)*      ***D06M 15/03*** *(2006.01)*

(21) Application number: **16190558.3**

(22) Date of filing: **26.09.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **24.09.2015   US 201562232323 P**
**23.09.2016   US 201615274811**

(71) Applicant: **Welspun India Limited**
**Mumbai 400013 (IN)**

(72) Inventors:
• **Goenka, Dipali**
**400013 Mumbai (IN)**
• **Goenka, Radhika**
**400013 Mumbai (IN)**
• **Palit, Subrata**
**400013 Mumbai (IN)**

(74) Representative: **Chapman, Alan Gareth**
**Avidity IP**
**Broers Building**
**Hauser Forum**
**21 JJ Thomson Avenue**
**Cambridge CB3 0FA (GB)**

(54) **PILLOW ARTICLE, TEXTILE MATERIAL, AND RELATED METHODS**

(57)    A textile article that includes a pillow case forming for a textile material includes a composition impregnated into the textile material.

Figure 1

**Description**

CROSS REFERENCE TO RELATED APPLICATIONS

This application claims the priority of U.S. Provisional Patent Application No. 62/232,323, filed September 24, 2015, which is incorporated by reference herein.

TECHNICAL FIELD

The present disclosure relates to a pillow article, a textile material for forming a pillow article, and method of making the textile material and pillow article.

BACKGROUND

Cosmetics and toiletry manufacturers have spent years developing face creams, lotions, and washes and other skin care products. Moisturizers, UV protection, and anti-aging products are sold to improve skin care health. Demand for such products is strong and shows no signs of abating. Consumers have a wide range of skin care options and competition among manufactures to deliver more robust and verifiable improvements to skin health is fierce. Manufacturers have responded with new compositions that have improved deposition of useful vitamins, minerals, and other herbal extracts onto the skin. Advances in delivery mechanisms, such as wipes, toilettes, applicators and other implements, have resulted in improvements as well. Implementing skin care compositions on textile materials is challenging. Compositions designed for direct application to skin are not directly translatable into textile dyeing and finishing processes. Balancing raw materials and processing variables to achieve end-use performance requirements is difficult and unpredictable.

SUMMARY

There is a need for a pillow article, a textile material, and a method of making same that can provide a skin care or wellness benefit. Accordingly, an embodiment of the present disclosure is an improved pillow article. The pillow article is configured to provide a skin care benefit and includes a pillow case. The pillow case includes upper and lower panels that define an internal space sized to receive a cushion member. The pillow case defines a first end, a second end spaced from the first end along a first direction, and opposed sides spaced apart with respect to each other along a second direction that is perpendicular to the first direction. At least one of the sides defines a seam that connects the upper panel to the lower panel. The pillow case is configured to be opened to provide access to the inner space. Further, each panel includes a plurality of warp yarns and a plurality of weft yarns interwoven with the plurality of warp yarns to define a woven fabric, the woven fabric including a composition disposed thereon that includes a hyaluronic acid component.

Another embodiment of the present disclosure is a woven fabric that includes cotton and silk fibers. The woven fabric includes a plurality of warp yarns with each warp yarn including a blend of cotton fibers and silk fibers, and a plurality of weft yarns that are interwoven with the plurality of warp yarns to define a woven structure. Each weft yarn includes at least cotton fibers. The warp and the weft yarns include a count in a range of about 10 Ne to about 80 Ne. The woven fabrics also include a composition impregnated into the woven fabric. The composition includes a hyaluronic acid component disposed on the woven fabric at a percent add-on of about 0.03 wt.% to about 10 wt.%.

Another embodiment of the present disclosure is a woven fabric that includes linen fibers. The woven fabric includes a plurality of warp yarns with each warp yarn including spun yarns that include either cotton fibers or linen fibers. The woven fabric also includes a plurality of weft yarns that are interwoven with the plurality of warp yarns to define a woven structure. Each weft yarn is a spun yarn that includes at least linen fibers. The warp and the weft yarns include a count in a range of about 10 Ne to about 80 Ne. The woven fabric also includes a composition impregnated into the woven fabric. The composition includes a hyaluronic acid component disposed on the woven fabric at a percent add-on of about 0.03 wt.% to about 10 wt.%.

Another embodiment of the present disclosure is a knitted fabric that includes a plurality of yarns formed into a knitted fabric that includes courses and wales of loops. Each yarn has a count in a range of about 10 Ne to about 80 Ne. The knitted fabric includes a composition impregnated thereon. The composition includes a hyaluronic acid component disposed on the woven fabric at a percent add-on of about 0.03 wt.% to about 10 wt.%. Another embodiment is a method of making a pillow article with such a knitted fabric.

Another embodiment of the present disclosure is a method of manufacturing a textile article. The method includes weaving a plurality of warp yarns with a plurality of warp yarns to define a woven fabric, wherein the warp and the weft yarns include a count in a range of about 10 Ne to about 80 Ne. The method includes printing a design onto a face of the woven fabric. The method includes applying a composition to the woven fabric. The composition includes a hyaluronic acid component at a concentration of about 0.03 wt.% to about 10 wt.%. The method includes curing the composition onto the woven fabric.

BRIEF DESCRIPTION OF THE DRAWINGS

The foregoing summary, as well as the following detailed description of illustrative embodiments of the present application, will be better understood when read in conjunction with the appended drawings. For the purposes of illustrating the present application, there is shown in the drawings illustrative embodiments of the disclosure. It should be understood, however, that the application is not limited to the precise arrangements and instrumentalities shown.

Figure 1 is a plan view of a pillow article, according to an embodiment of the present disclosure.

Figure 2 is cross-sectional view of the pillow article taken along line 2-2 in Figure 1.

Figure 3 is schematic process flow diagram for manufacturing the pillow article illustrated in Figure 1.

DETAILED DESCRIPTION OF ILLUSTRATIVE EMBODIMENTS

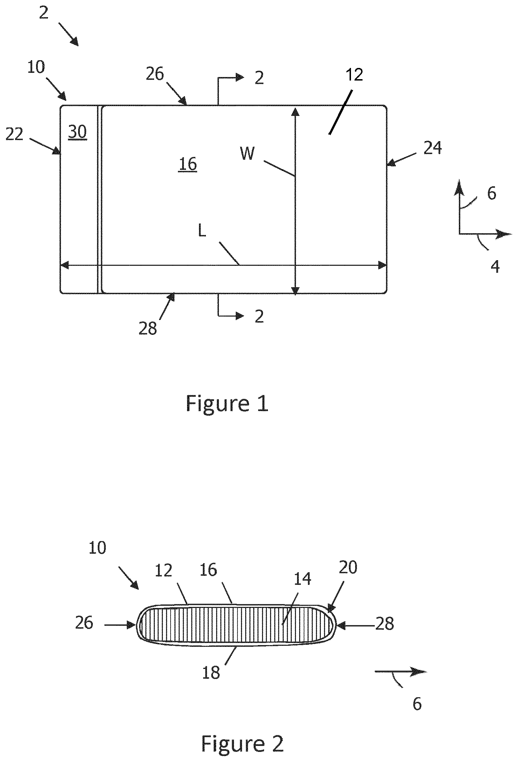

Embodiments of the present disclosure include a textile article 2 illustrated in the form of a pillow 10. Figures 1 and 2 illustrate a pillow 10 that includes a pillow case 12 and a cushion member 14 located inside the pillow case 12. The pillow 10 and the pillow case 12, in particular, may include one or more functional agent selected to as skin care or skin wellness promoters. It is believed that the described functional agent is deposited onto the consumer's skin during use. Repeated usage may result in therapeutic benefit to the skin, such as reduction in skin wrinkles, limitation of free radical damage, and firming skin cells in the epidermal and dermal skin layers. The described functional agent can hydrate skin and increase oxygen content in the skin.

Continuing with Figures 1 and 2, the pillow case 12 includes an upper panel 16 and a lower panel 18 that define an inner space 20 sized to receive a cushion member 14. The pillow case includes a first end 22, a second end 24 spaced from the first end 22 along a first direction 4, and opposed sides 26 and 28 spaced apart with respect to each other along a second direction 6 that is perpendicular to the first direction 4. A block hem 30 is included along the first end 22. The pillow case 12 can be manufactured such that the upper and lower panels 16 and 18 are a single monolithic pieces seamed along one of the sides (26 or 28) and along second end 24. Alternatively, the upper and lower panels 16 and 18 are separate components joined together along sides 26 and 28 and end 24.

As shown Figure 1, the pillow case 10 defines a length L that extends from the first end 22 to the second end 24 along the first direction 4, and a width W (when cushion member is removed from the pillow case) that extends from side 26 to the side 28 along the second direction 6. In accordance with the illustrated embodiment, the pillow case 10 has a length L that is greater than the width W to define a rectangular shaped pillow case. However, the pillow case 10 is not limited to the shape illustrated. For instance, in other embodiments, the length L and width W can be substantially the same to define a square shaped pillow case. In further embodiments, the pillow case 10 has a length L that is smaller than the width W. However, the pillow case 10 is not limited to rectangular or square shapes.. For instance, the pillow case 10 can be circular, triangular (a shape with three even or differently sized sides), trapezoidal, or a shape with five or more sides.

As shown in Figure 2, the pillow case 10 is configured to provide access to interior space 20 so that the cushion member 14 can be removed as needed. As illustrated, the first end 22 is configured to provide access to the inner space 20 so that the cushion member 14 can be removed. In one example, the first end 22 is open to provide access to interior space 20. In another example,, the first end 22 can include a closure device, such as a zipper, buttons, snap, toggles or the like. It should be appreciated the access to the interior space 20 can be provided along any portion of the ends 22 and 24, sides 26 and 28, or along panels 16 and 18. For instance, a decorative pillow may provide access at side 26 or across the lower panel 18.

The cushion member 14 can be any type of insert or structure that provides cushion and resilience to the pillow 10. For instance, the cushion member can be a bag of fiber fill, down feathers, or ground foam particles. The cushion member 14 can also be a monolithic insert. The monolithic insert may be latex, memory foam, open or closed cell foams a batting material, or any combination thereof. A batting material is lofty assembly of fibers that may be either mechanical, chemically, or thermally bonded together.

The pillow case 10 is formed from a textile material that includes a composition of one or more functional agents described above. As illustrated, the textile material is a woven fabric that includes a plurality of warp yarns and a plurality of weft yarns interwoven with the plurality of warp yarns to define a woven structure. The woven fabric may be defined by a number of different woven structures. Exemplary woven structures include, but are not limited to, satins (e.g. 5/1 satins, 4/1 satin, 4/1 satin base strip, 4/1 stain swiss dot, 4/1 down jacquard), 1x1 plain weave, basket weaves, 2x1 rib weave, 2x2 rib weave, or 3x1 rib weave, and twill weaves. In one example, the satin woven structures are preferred, such as the 4/1 satin, etc.

The warp yarns can be any type of spun yarn structure. For example, the warp yarns can be ring spun yarns, open end yarns, or rotor spun yarns, or filaments. In another embodiment, the warp yarns can be Hygrocotton ® brand yarns marketed by Welspun India Limited. Furthermore, yarns can be formed as disclosed in U.S. Patent No. 8,833,075, entitled "Hygro Materials for Use In Making Yarns And Fabrics," (the 075 patent). The 075 patent is incorporated by reference into present disclosure. Preferred warp yarns are ring spun.

The warp yarns can be formed from any number of fiber types. For instance, the warp yarns include natural fibers, synthetic fiber yarns, or blends of natural and synthetic fibers. Preferred natural fibers include cotton, silk and linen. However, other natural fibers could be used, such as flax, bamboo, hemp, wool, and the like. Preferred synthetic fibers are those fibers that result in fabric structures with good hand, drape, and softness. Preferred synthetic fibers may include rayon fibers (e.g. Modal, Lyocell). In some examples, thermoplastic fibers could be used blend yarns, such as polyethylene terephthalate (PET) fiber, polylactic acid (PLA) fiber, polypropylene (PP) fibers, polyamide fibers, and microfiber staple fibers.

The warp yarns can be single fiber yarns or blended yarns. In one example, the warp yarns are formed from cotton fibers. In another example, the warp yarns are blended yarns that include cotton fibers and silk fibers. For instance, such blended yarns include about 50 wt.% to about 80 wt.% cotton fibers and about 20 wt.% to about 50 wt.% silk fibers. In other words, the blend yarns include about 50, 55, 60, 65, 70, 75 to about 80 wt.% of cotton fibers to about 20, 25, 30, 35, 40, 45, to about 50 wt.% of silk fibers. In yet another example, the warp yarns are blended yarns that include about 70 wt.% cotton fibers and about 30 wt.% silk fibers. In another example, the warp yarns are blended yarns that include cotton fiber and rayon fibers. In another example, the warp yarns are formed from linen fibers.

The modifier "about" as used herein discloses the range defined by the absolute values of the two endpoints. For example, the expression "about 2 to about 4" also discloses the range "from 2 to 4." When used to modify a single number, the term "about" may refer to plus or minus 10% of the indicated number and includes the indicated number. For example, "about 10%" may indicate a range of 9% to 11%, and "about 1" means 0.9 to 1.1.

The warp yarns have a range of counts for the yarn types and fibers as described above. For instance, the warp yarns can have count of about 10 Ne to about 80 Ne. In other words, the warp yarns can have a count of about 10, 15, 20, 25, 30, 35, 40, 45, 50, 55, 60, 65, 70, 75, to about 80 Ne. In one example, the warp yarns have a count of about 10 Ne. In another example, the warp yarns have a count of about 14 Ne. In another example, the warp yarns have a count of about 20 Ne. In another example, the warp yarns have a count of about 24 Ne. In another example, the warp yarns have a count of about 30 Ne. In another example, the warp yarns have a count of about 40 Ne. In another example, the warp yarns have a count of about 50 Ne. In another example, the warp yarns have a count of about 60 Ne. In another example, the warp yarns have a count of about 70 Ne. In another example, the warp yarns have a count of about 80 Ne. In addition, the warp yarns can be plied yarns. As used herein, the term "plied" refers to a yarn having two or more strands and is abbreviated "ply". In one example, the natural fiber warp yarn is 2-ply yarn. In another example, the warp yarns yarn is a 3-ply yarn.

The weft yarns can be any type of spun yarn structure. For example, the weft yarns can be ring spun yarns, open end yarns, rotor spun yarns, or filaments. In another embodiment, the weft yarns can be Hygrocotton ® brand yarns.

The weft yarns can be formed from any number of fiber types. For instance, the weft yarns include natural fibers, synthetic fiber yarns, or blends of natural and synthetic fibers. In some embodiments, the natural fibers include cotton, silk and linen. However, other natural fibers could be used, such as flax, bamboo, hemp, wool, and the like. The synthetic fibers are those fibers that result in fabric structures with good hand, drape, and softness. In some embodiments, the synthetic fibers include rayon fibers (e.g. Modal, Lyocell). In other embodiments, thermoplastic fibers may be included in blended yarns. Thermoplastic fibers include, without limitation, polyethylene terephthalate (PET) fiber, polylactic acid (PLA) fiber, polypropylene (PP) fibers, polyamide fibers, and microfiber staple fibers.

The weft yarns may be single fiber yarns or blended yarns. In one embodiment, the weft yarns include cotton fibers. In other embodiment, the weft yarns are blended yarns that include cotton fibers and silk fibers. For instance, the blended yarns may include about 50 wt.% to about 80 wt.% cotton fibers and about 20 wt.% to about 50 wt.% silk fibers. In other words, the blended yarns may include about 50, 55, 60, 65, 70, 75, to about 80 wt.% cotton fibers and about 20, 25, 30, 35, 40, 45 to about 50 wt.% silk fibers. In one example, the weft yarns are blended yarns that include about 70 wt.% cotton fibers and about 30 wt.% silk fibers. In another example, the weft yarns include cotton and rayon (e.g. lycocell) fibers. For instance, such weft yarns contain about 40 wt.% to about 80 wt.% rayon fibers and about 20 wt.% to about 60 wt.% cotton fibers. In other words, the weft yarns contain about 40, 45, 50, 55, 60, 65, 70, 75 to about 80 wt.% rayon fibers and about 20, 25, 30, 35, 40, 45, 50, 55 to about 60 wt.% cotton fibers. In one example, the blended yarns include about 40 wt.% cotton fibers and about 60 wt.% rayon fibers. In another example, the weft yarns are formed from linen fibers.

The weft yarns have a range of counts for the yarn types and fibers used as described above. For instance, the weft yarns can have count of about 10 Ne to about 80 Ne. In other words, the weft yarns have a count of about 10, 15, 20, 25, 30, 35, 40, 45, 50, 55, 60, 65, 70, 75 to about 80 Ne. In one example, the weft yarns have a count of about 10 Ne. In another example, the weft yarns have a count of about 14 Ne. In another example, the weft yarns have a count of about 20 Ne. In another example, the weft yarns have a count of about 24 Ne. In another example, the weft yarns have a count of about 30 Ne. In another example, the weft yarns have a count of about 40 Ne. In another example, the weft yarns have a count of about 50 Ne. In another example, the weft yarns have a count of about 60 Ne. In another example, the weft yarns have a count of about 70 Ne. In another example, the weft yarns have a count of about 80 Ne. In addition, the weft yarns can be plied yarns. In one example, the natural fiber weft yarn is 2-ply yarn or greater. In another example, the weft yarns yarn is a 3-ply yarn.

In alternative embodiments, the textile material may be a knit fabric. The knit fabric may be a weft knit, such as single jersey knit fabric, a double knit, rib knit, or any other type of weft knitted fabric. The knit fabric may alternatively be a warp knit, such as a tricot or rachel warp knitted fabric. The knit fabric may include any one of the warp or weft yarns described above with respect to the woven fabric, including fiber type, yarn type and yarn counts. In some embodiments, the knit fabric can include yarns with cotton fibers; yarns that include cotton fibers and silk fibers; yarns that include cotton and rayon (e.g. lycocell) fibers; or yarns formed from linen fibers.

The composition of functional agent applied to the woven fabric may include a hyaluronic acid component, an aloe vera component, a vitamin E component, and an antimicrobial component. Each component will be discussed below. In accordance with the illustrated embodiment, the functional agent includes at least one of a hyaluronic acid, aloe vera extract, vitamin E emulsion, an antimicrobial agent, and a catalyst. It should be appreciated that additional functional agents could be used, such as softeners and the like.

The hyaluronic acid component helps to firm skin cells, acts as an antioxidant and can increase further active oxygenation in skin cells. Hyaluronic acid is a substance that is naturally present in the human body. Typically, hyaluronic acid it is found in the highest concentrations in fluids in the eyes and joints. The hyaluronic acid that is used as medicine may be extracted from rooster combs or made by bacteria in the laboratory. Hyaluronic acid has been used for joint disorders, oral or injected, certain eye surgeries including cataract removal, corneal transplantation, and repair of a detached retina and other eye injuries. It is injected into the eye during the procedure to help replace natural fluids. Further, hyaluronic acid is also used as a filler in plastic surgery. Utilizing hyaluronic acid in applied the woven fabric, or pillow case, can result moisturizing and film-forming properties, wrinkle reduction, promote skin self hydration.

The hyaluronic acid component as disclosed herein may be used in a powder form and applied after dilution. Typically, the hyaluronic acid dilution has a pH of about 6 to about 8. It should be appreciated that hyaluronic acid and salts thereof could be used as described herein. Examples of salts of hyaluronic acid include, without limitation, sodium hyaluronate, potassium hyaluronate. Table 1 below illustrates some exemplary properties of hyaluronic acid that are suitable for use in the composition as described herein.

**Table 1**

| Property | Version A | Version B |
|---|---|---|
| Appearance | White or almost white powder / fibrous aggregate | White or almost white powder / fibrous aggregate |
| pH | about 5.0 to about 8.5 | about 5.0 to about 8.5 |
| Transparency | about 85 wt.% to about 100 wt.%, such as about 99 wt.% | about 85 wt.% to about 100 wt.%, such as about 99 wt.% |
| Loss on drying | about 5 wt.% to about 15 wt.%, such as about 10 wt. % | about 5 wt.% to about 15 wt.%, such as about 10 wt.% |
| Molecular Weight | about $1.0 \times 10^6$ KDa to about $1.8 \times 10^6$ KDa | less than about $1.0 \times 10^6$ KDa |
| Protein | about 0.1 wt.% to about 0.5 wt.%, such as about 0.3 wt. % | about 0.1 wt.% to about 0.5 wt.%, such as about 0.3 wt.% |
| Heavy metals | about 5 to about 50 ppm, such as about 20 ppm | about 5 to about 50 ppm, such as about 20 ppm |
| Glucuronic acid | about 35 wt.% to about 50 wt.%, such as about 44 wt.% | about 35 wt.% to about 50 wt.%, such as about 44 wt.% |

As used herein, the molecular weight for the hyaluronic acid refers to the weight average molecular weight ($M_w$). The $M_w$ recited herein is determined by gel permeation chromatography using the following equation, where $M_i$ is the molecular weight of a chain and $N_i$ is the number of chains of that molecular weight.

$$M_w = \frac{\sum N_i M_i^2}{\sum N_i M_i}$$

The term "aloe vera" as used herein refers to one or more of the phytochemicals in *aloe vera* leaves. The phytochemicals may be utilized may be used as part of the aloe vera leaf or extracted therefrom. Such phytochemicals include, without limitation, acetylated mannans, polymannans, anthraquinones such as emodin, anthraquinone C-glycosides, anthrones, and lectins. The aloe vera component may also be an emulsion of an aloe vera extract. In other forms, the aloe vera component may be an emulsion of aloe vera extract and Jojoba oil. Accordingly, the aloe vera component may include other agents, such as jojoba oil.

The vitamin E component is lipid-soluble vitamin in the form of $\alpha$-tocopherol ("alpha-tocopherol"). In some embodiments, the vitamin component may be in form of an emulsion of alpha-tocopherol. The emulsion can include a polyurethane microcapsule wall material and alpha-tocopherol oil as the core.

The antimicrobial components include agents that can limit bacterial growth on the woven fabric. The antimicrobial agent can be silver (such as colloidal silver and formulations containing silver salts), nano-silver (such as nanoparticles of silver which are in the range of about 1 to about 100 nm in size), quaternary ammonium salt, or other antibacterial agents. In one example, the antimicrobial agent is a silver compound. In another example, the antimicrobial agent is a nano-silver compound.

When the composition is applied to the fabric as further explained below, each component can have a percent (%) add-on within the ranges summarized in table 2 below. Percent add-on is the product of the concentration of the component and the wet pick up (WPU) during the application process. The concentration is obtained by dividing the total weight of the component by the total weight of the composition (including water). The percent add-on values in table 1 are based on the range of component concentration and WPU for applying the composition to the woven fabric as further detailed below. Table 2 illustrates an example where each functional agent is applied to the woven fabric.

**Table 2**

| Component | % wt.Add-On |
|---|---|
| Hyaluronic Acid | about 0.03 wt.% to about 10 wt.% |
| Aloe Vera | about 0.03 wt.% to about 15 wt.% |
| Vitamin E | about 0.03 wt.% to about 15 wt.% |
| Antibacterial | about 0.03 wt.% to about 15 wt.% |
| Catalyst | about 0.03 wt.% to about 15 wt.% |

In some embodiments, the composition applied to the fabric may include just one of the components listed Table 2, water, and nominal amounts of process aids, such as a catalyst (if needed), buffers, surfactants, or other agents may be used even when just one of the components is used. For example, the composition applied to the fabric may include the hyaluronic acid at a percent add-on of about 0.03 wt.% to about 10 wt.%. In other words, the composition applied to the fabric may include the hyaluronic acid at a percent add-on of about 0.03, 0.05, 1, 1.5, 2, 2.5, 3, 3.5, 4, 4.5, 5, 5.5, 6, 6.5, 7, 7.5, 8, 8.5, 9, 9.5 to about 10 wt.%. In another example, the composition applied to the fabric may include the aloe vera component at a percent add-on of about 0.03 wt.% to about 15 wt.%. In other words, the composition applied to the fabric may include the aloe vera component at a percent add-on of about 0.03, 0.05, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5 to about 15 wt.%. In another example, the composition applied to the fabric may include the vitamin E component at a percent add-on of about 0.03 wt.% to about 15 wt.%. In other words, the composition applied to the fabric may include the vitamin E component at a percent add-on of about 0.03, 0.05, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5 to about 15 wt.%. In another example, the composition applied to the fabric may include the antimicrobial component at a percent add-on of about 0.03 wt.% to about 15 wt.%. In other words, the composition applied to the fabric may include the anti-microbial component at a percent add-on of about 0.03, 0.05, 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5 to about 15 wt.%.

In other embodiments of the present disclosure, the composition applied to the fabric may include just two of the components listed Table 2, water, and nominal amounts of other process aids. In one example, the composition applied to the fabric may include the hyaluronic acid component and the aloe vera component. In another example, the composition applied to the fabric may include the hyaluronic acid component and the vitamin E component. In another example, the composition applied to the fabric may include the hyaluronic acid component and the antibacterial component. In another example, the composition applied to the fabric may include the vitamin E component and the aloe vera component. In another example, the composition applied to the fabric may include the vitamin E component and the antibacterial component. In another example, the composition applied to the fabric may include the aloe vera component and the antibacterial component. In all examples in this paragraph, for each respective component, the percent add-on is similar to the percent add-on summarized in Table 2 above.

In one example, the composition of the functional agents applied to woven fabric includes the hyaluronic acid component, the aloe vera component, and the vitamin E component. In another example, the composition includes the hyaluronic acid component, the aloe vera component, and the antibacterial component. In another example, the composition includes the hyaluronic acid component, the vitamin E component, and the antibacterial component. In another example, the composition includes the aloe vera component, the vitamin E component, and the antibacterial component. In all examples in this paragraph, for each respective component, the percent add-on is similar to the percent add-on summarized in Table 1 above.

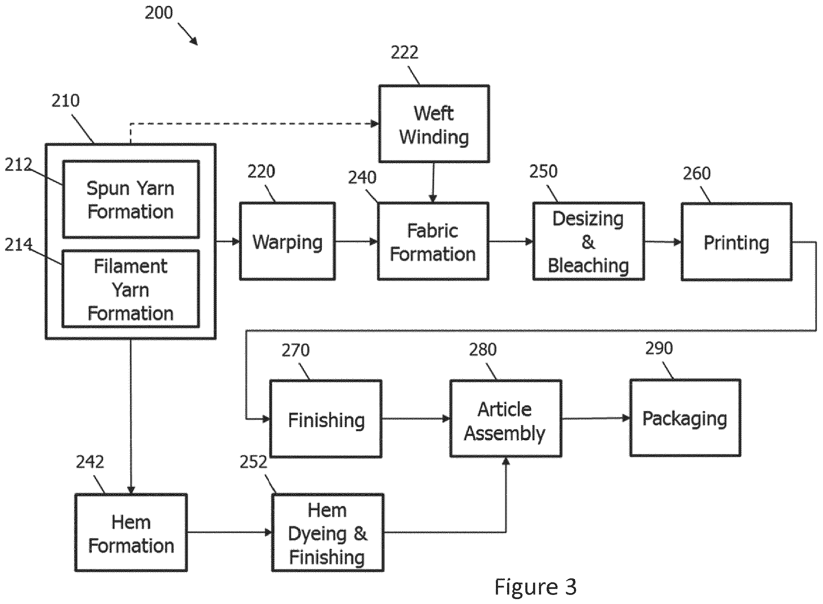

Turning to Figure 3, a method of making a textile article according to an embodiment of the disclosure is illustrated. The method 200 includes yarn formation steps 210 for warp and weft yarns. Yarn formation 210 for the warp yarns can include staple yarn formation or spinning 212 and filament yarn formation 214 (where applicable). Staple yarn formation 212 may utilize any number of yarn formation systems and sub-systems. For instance, staple yarn formation may include bale opening, carding, optionally combing, drafting, roving, and yarn spinning (yarn spinning processes are not illustrated but are contemplated) to the desired count and twist level. In some cases, the warp yarns can be plied into 2-ply, 3-ply, or 4-ply configurations. After yarn spinning, the warp yarns are wound into the desired yarn packages for the warping step 220. In one example, ring spinning is the spinning system. However, the warp yarns can be formed using open end spinning systems or rotor spun spinning systems. Furthermore, the spinning system may include methods to form the Hygrocotton ®, as disclosed in the 075 patent. The 075 patent is incorporated by reference into present disclosure.

The filament formation forms continuous filament yarns. During filament formation, polymer resins (such as PET, PLA, and PP) are melted and extruded through orifices at temperatures that approach the polymer melting temperature ($T_m$). From the orifices, the filaments may be tensioned by passing over one or more godets before being wound onto yarn packages. Additional bulking or texturizing steps may be included to increase the bulk and impart false twist to the yarns as is known in the textile arts.

During yarn formation 210, the weft yarns may be formed with similar fiber types and using the same or similar yarn spinning systems used to form the warp yarns. As needed, the weft yarns may be plied in 2-ply, 3 ply, or 4-ply configurations. Following weft yarn spinning, the weft winding step 222 prepares wound packages of weft yarns. The wound packages are then staged for weft insertion during fabric formation steps discussed further below.

As noted above, warping step 220 follows the yarn formation step 210. In the warping step 220, warp yarn ends are removed from their respective yarn packages, arranged in a parallel form, and wound onto a warp beam. The warping step 220 also includes a sizing step where a typical sizing agent is applied to each warp yarn to aid in fabric formation. The warping step 220 results in a warp beam of warp yarns prepared for weaving. The warp beam can be positioned on a mounting arm of a weaving loom so that the warp yarns can be drawn through the loom components, as further described below.

Continuing with Figure 3, following the warping step 220, a weaving step 240 forms a woven fabric using a weaving loom. More specifically, in the weaving step 240, the warp yarns are drawn-in (not shown, but contemplated) through various components of a weaving loom, such as drop wires, heddle eyes attached to a respective harness, reed and reed dents, in a designated order as is known in the art. After drawing-in is complete, the weaving step 240 proceeds through formation phase. The formation phase creates shed with the warp yarns that the weft or picks can be inserted through across the width direction of the machine to create the desired woven fabric construction. For instance, shedding motions can include cam shedding, dobby shedding, or jacquard shedding motions, each of which can cause the selective raising and lowering of warp ends to create an open shed for weft insertion.

During the formation phase of the weaving step 240, weft yarns are interwoven with the warp yarns to define the woven design constructions. Exemplary fabric woven constructions can include but are not limited to, satins (e.g. satin 5/1, satin 4/1 satin; 4/1 satin base strip; 4/1 stain swiss dot; 4/1 down jacquard; 5/1 satins); 1x1 plain weave; basket weaves; 2x1 rib weave; 2x2 rib weave; or 3x1 rib weave; and twill structures. In one example, the satin woven structures are preferred, such as the 4/1 satin, etc. The formation phase can utilize different weft insertion techniques, includes air-jet, rapier, or projectile type weft insertion techniques. The weaving step 240 can further include weaving one or more selvedge edges along a length L of the woven fabric.

The weaving step 240 can form woven fabrics having any number of different fabric constructions. In one example, the woven fabrics can be formed to include about 10 to about 100 warp ends/cm. In other words, the woven fabrics can be formed to include about 10, 15, 20, 25, 30, 35, 40, 45, 50, 55, 60, 65, 70, 75, 80, 85, 90, 95 to about 100 warp ends/cm. In some embodiments, the woven fabrics include about 40 and 80 warp ends/cm. The weft or pick density can be about 10 to about 60 picks/cm. In other words, the weft or pick density can be about 10, 15, 20, 25, 30, 35, 40, 45, 50, 55 to about 60 picks/cm. In some embodiments, the pick density is about 20 picks/cm to about 40 picks/cm.

Following weaving step 240, the woven fabric passes through desizing and bleaching step 250. Desizing may be accomplished with typical enzymes used to remove sizing material from the warp ends. Bleaching may include typical bleaching agents known in the art, such as hydrogen peroxide bleaching. Step 250 may include singing the fabric.

Next, a printing step 260 applies a design on the face of the woven fabric The design is printed so that the design is displayed on the upper and lower panels of the pillow case 12 when assembled. The printing step 260 initiates with a preparation step that includes padding the woven fabric with a paste, such as a paste containing sodium alginate and alkali, at a typical WPU and utilizing typical concentrations which are known in the art. After padding the paste onto the woven fabric, a digital printer prints a design the fabric using reactive inks or dyestuffs. After printing, the printed, woven fabric is steamed and washed. The steaming and washing steps remove the printing gums and any unfixed dyestuffs.

After printing step 260, a finishing step 270 applies a composition including one or more of the functional agents to the woven fabric. The finishing step 270 includes multiple steps including a) an application step, b) a drying step, and c) a curing step where the functional agents applied to the woven fabric are cured.

As noted above, the application step includes applying the composition to the woven fabric. A padding operation may be used to apply the composition to the woven fabric. In one particular example, the composition applied to fabric is summarized in Table 3 below.

**Table 3**

| Component | g/L | % Concentration | % on weight of bath (OWB) |
|---|---|---|---|
| Hyaluronic Acid | 0.5 | 0.05 | 0.05 |
| Aloe Vera | 40 | 3.63 | 4 |
| Vitamin E | 20 | 1.82 | 2 |
| Antibacterial | 30 | 2.72 | 3 |
| Catalyst | 10 | 0.91 | 1 |
| Water | 1000 | 90.87 | 100 |

During padding, a targeted wet pick-up (WPU) is about 60 wt.% to about 160 wt.% for a targeted add-on levels. It should be appreciated that the WPU ranges from about 60, 70, 80, 90, 100, 110, 120, 130, 140, or 150 to about 160%WPU. In some embodiments, the targeted WPU is about 90 % to about 110 %. As used herein, WPU is equal to the weight of the solution picked up x 100 divided by the dry weight of the fabric just prior to application step. The composition listed above has a pH of about 5.0 to about 6.0 during padding. It should be appreciated that the values listed in Table 3 are exemplary and should not be considered a limitation herein. For example, each component may be used a different g/L depending on the specific fiber and fabric combination. Thus, the weight of each component per liter of solution may be increased or decreased as needed.

Accordingly, embodiments of the present disclosure can include compositions with the hyaluronic acid component at a concentration of about 0.03 wt.% to about 10 wt.%; the aloe vera component at a concentration of about 0.03 wt.% to about 15 wt.%; the vitamin E component at a concentration of about 0.03 wt.% to about 15 wt.%; the antimicrobial component at a concentration of about 0.03 wt.% to about 15 wt.%; and the catalyst at a concentration of about 0.03 wt.% to about 15 wt.%.

Next, the drying step of finishing 270 removes excess moisture from the woven fabric and composition. During the drying step, the woven fabric is advanced through a heating machine at a rate of about 2.0 meters/min to about 30 meters/min, which varies based on number heating zones. In other words, the woven fabric is advanced through a heating machine at a rate of about 2, 5, 10, 15, 20, 25, to about 30 meters/min. The heating machine may be set to expose the fabric to at about 140 °C for a period of time. The drying step may include convection, heated steam, infrared, hot air, surface rolls, hot oil can, through-air ovens and the like.

After drying, the components of the composition on the fabric are cured. The curing step includes exposing the woven fabric to thermal energy for a period of time that is sufficient to induce cure in the composition components. Curing may include exposing the woven fabric to heated air, a heated surface (e.g. a calendar roll), or an infrared heat source. In one example, the woven fabric is exposed to about 150 °C for about 45 to about 60 seconds, which could be about 2.0 meters/min up to about 30 meters/min based on the number heating zones. The heating machine could also dry and cure the fabric.

Following the curing step, the fabric is assembled into the article in step 280. As illustrated, the assembly step 280 includes cutting the woven fabric size for use a pillow case. Following cutting, additional edge binding or a hem can be applied to finish the cut edges. In one example, a block hem 30 can be formed in step 242 using a typical fabric formation processes known in the art. The block hem 30 can be dyed separately using reactive dyes as in known in the art and finished in step 252. The block hem 30 is then used in article assembly step 280 to finish the pillow case 10. In some cases, the cutting and sewing may include inserting the cushioning member 14 in the completed pillow case 10. However, the process does not require completed pillows with a cushioning member 14. Rather, the method 200 can result in the pillow case 10 formed as described herein.

After the cutting step, a packaging step 290 places the pillow case (or completed pillow) in suitable packaging for shipment.

Tables 4-13 below illustrate exemplary fabrics formed as described herein. The examples should not be considered limiting. For each example discussed below, the composition illustrated in tables 2 and 3 could be applied to the fabric as described above in method 200.

**Table 4 Example 1**

| | |
|---|---|
| Weave | 4/1 Satin |
| Warp Yarns | Ring Spun, 60Ne, 70 wt.% cotton/30 wt.% silk |
| Warp End Density | 180 ends/inch (70.8 ends/cm) |
| Weft Yarns | Ring Spun, 60Ne, 70 wt.% cotton/30 wt.% silk |
| Weft Density | 74 picks/inch (29.1 picks/cm) |

**Table 5 Example 2**

| | |
|---|---|
| Weave | 4/1 Satin |
| Warp Yarns | Ring Spun, 60Ne, 70 wt.% votton/30 wt.% silk |
| Warp End Density | 180 ends/inch (70.8) ends/cm |
| Weft Yarns | Ring Spun, 60Ne, 60 wt.% lyocell/40 wt.% cotton |
| Weft Density | 74 picks/inch (29.1 picks/cm), double pick insertion |

**Table 6 Example 3**

| | |
|---|---|
| Weave | 4/1 Satin |
| Warp Yarns | 40 lea, linen fibers |
| Warp End Density | 52 ends/inch (20.4 ends/cm) |
| Weft Yarns | 40 lea (14 Ne) linen fibers |
| Weft Density | 52 picks/inch (20.4 picks/cm) |

**Table 7 Example 4**

| | |
|---|---|
| Weave | 4/1 Satin |
| Warp Yarns | 24 Ne cotton fibers |
| Warp End Density | 52 ends/inch (20.4 ends/cm) |
| Weft Yarns | 14 lea, linen fibers |
| Weft Density | 52 picks/inch (20.4 picks/cm) |

**Table 8 Example 5**

| Weave | Plain Weave |
|---|---|
| Warp Yarns | Ring Spun, 60Ne, 70 wt.% cotton/30 wt.% silk |
| Warp End Density | 180 ends/inch (70.8 ends/cm) |
| Weft Yarns | Ring Spun, 60Ne, 70 wt.% cotton/30 wt.% silk |
| Weft Density | 74 picks/inch (29.1 picks/cm) |

**Table 9 Example 6**

| Weave | Plain Weave |
|---|---|
| Warp Yarns | Ring Spun, 60Ne, 70 wt.% cotton/30 wt.% silk |
| Warp End Density | 180 ends/inch (70.8) ends/cm |
| Weft Yarns | Ring Spun, 60Ne, 60 wt.% rayon/40 wt.% cotton |
| Weft Density | 74 picks/inch (29.1 picks/cm), double pick insertion |

**Table 10 Example 7**

| Weave | Plain Weave |
|---|---|
| Warp Yarns | 40 lea, linen fibers |
| Warp End Density | 52 ends/inch (20.4 ends/cm) |
| Weft Yarns | 40 lea (14 Ne) linen fibers |
| Weft Density | 52 picks/inch (20.4 picks/cm) |

**Table 11 Example 8**

| Weave | 4/1 Satin and plain weave |
|---|---|
| Warp Yarns | 24 Ne cotton fibers |
| Warp End Density | 52 ends/inch (20.4 ends/cm) |
| Weft Yarns | 14 lea, linen fibers |
| Weft Density | 52 picks/inch (20.4 picks/cm) |

**Table 12 Example 9**

| Knit | Rib knit |
|---|---|
| Yarns | Ring Spun, 60Ne, 70 wt.% cotton/30 wt.% silk |
| Course Count | 20 |
| Wale Count | 20 |

**Table 13 Example 10**

| Knit | Jersey |
|---|---|
| Yarns | Ring Spun, 60Ne, 70 wt.% cotton/30 wt.% silk |
| Course Count | 30 |

(continued)

| Knit | Jersey |
|---|---|
| Wale Count | 30 |

**Table 14 Example 11**

| Knit | Jersey |
|---|---|
| Yarns | Ring Spun, 60Ne, 60 wt.% rayon/40 wt.% cotton |
| Course Count | 30 |
| Wale Count | 30 |

Each exemplary fabric summarized in tables 4-13 includes a printed design and a composition that includes the hyaluronic acid component, the aloe vera component, the vitamin E component, and the antimicrobial component at the concentrations summarized in Table 3.

While the disclosure is described herein using a limited number of embodiments, these specific embodiments are not intended to limit the scope of the disclosure as otherwise described and claimed herein. The precise arrangement of various elements and order of the steps of articles and methods described herein are not to be considered limiting. For instance, although the steps of the methods are described with reference to sequential series of reference signs and progression of the blocks in the figures, the method can be implemented in a particular order as desired.

PARAGRAPHS OF THE INVENTION

A pillow article configured to provide a skin care benefit, the pillow article comprising:

a pillow case including upper and a lower panels that define an internal space sized to receive a cushion member, the pillow case defining a first end, a second end spaced from the first end along a first direction, and opposed sides spaced apart with respect to each other along a second direction that is perpendicular to the first direction, and at least one of the sides defining a seam that connects the upper panel to the lower panel, the pillow case is configured to be opened to provide access to the inner space, each panel including a plurality of warp yarns and a plurality of weft yarns interwoven with the plurality of warp yarns to define a woven fabric, the woven fabric including a composition disposed thereon that includes a hyaluronic acid component.

The pillow article of paragraph 60, wherein the composition includes at least one of: an aloe vera component, a vitamin E component, and an antimicrobial component.

The pillow article of paragraph 61, wherein the composition includes the hyaluronic acid component, the aloe vera component, the vitamin E component, and the antimicrobial component.

The pillow article of paragraph 62, wherein the hyaluronic acid component is disposed on the fabric at a percent add-on of about 0.03 wt.% to about 10 wt.%.

The pillow article of paragraph 62, wherein the aloe vera component is disposed on the fabric at a percent add-on of about 0.03 wt.% to about 15 wt.%.

The pillow article of paragraph 62, wherein the vitamin E component is disposed on the fabric at a percent add-on of about 0.03 wt.% to about 15 wt.%.

The pillow article of paragraph 62, wherein the antimicrobial component is disposed on the fabric at a percent add-on of about 0.03 wt.% to about 15 wt.%.

The pillow article of paragraph 62, wherein the catalyst is disposed on the fabric at a percent add-on of about 0.03 wt.% to about 15 wt.%.

The pillow article of paragraph 60, wherein the warp yarns are blended yarns that include cotton and silk fibers.

The pillow article of paragraph 68, wherein the blended yarns include about 50 wt.% to about 80 wt.% cotton fibers and about 20 wt.% to about 50 wt.% silk fibers

The pillow article of paragraph 69, wherein the blended yarns include about 70 wt.% cotton fibers and about 30 wt.% silk fibers.

The pillow article of paragraph 60, wherein the weft yarns are blended weft yarns that include cotton and silk fibers.

The pillow article of paragraph 71, wherein the blended yarns include about 50 wt.% to about 80 wt.% cotton fibers and about 20 wt.% to about 50 wt.% silk fibers

The pillow article of paragraph 72, wherein the blended yarns include about 70 wt.% cotton fibers and about 30 wt.% silk fibers.

The pillow article of paragraph 60, wherein the weft yarns are blended weft yarns that include cotton and rayon fibers.

The pillow article of paragraph 74, wherein the blended yarns include about 20 wt.% to about 60 wt.% cotton fibers and about 40 wt.% to about 80 wt.% rayon fibers.

The pillow article of paragraph 75, wherein the blended yarns include about 40 wt.% cotton fibers and about 60 wt.% rayon fibers.

The pillow article of paragraph 60, wherein either the warp yarns and the weft yarns are formed from linen fibers or the warp yarns are formed from cotton fibers and the weft yarns are formed from linen fibers.

A woven fabric, comprising:

a plurality of warp yarns, each warp yarn including a blend of cotton fibers and silk fibers;
a plurality of weft yarns that are interwoven with the plurality of warp yarns to define a woven structure, each weft yarn including at least cotton fibers, wherein the warp and the weft yarns include a count in a range of about 10 Ne and to about 80 Ne; and
a composition impregnated into the woven fabric, the composition including a hyaluronic acid component disposed on the woven fabric at a percent add-on of about 0.03 wt.% to about 10 wt.%.

The woven fabric of paragraph 78, wherein the composition further includes at least one of:

an aloe vera extract component disposed on the woven fabric at a percent add-on of about 0.03 wt.% to about 15 wt.%;
a vitamin E component disposed on the woven fabric at a percent add-on of about 0.03 wt.% to about 15 wt.%; and
antimicrobial component disposed on the woven fabric at a percent add-on of about 0.03 wt.% to about 15 wt.%.

The woven fabric of paragraph 78, wherein the warp yarns include about 50 wt.% to about 80 wt.% cotton fibers and about 20 wt.% to about 70 wt.% silk fibers.

The woven fabric of paragraph 80, wherein the warp yarns include about 70 wt.% cotton fibers and about 30 wt.% silk fibers.

The woven fabric of paragraph 78, wherein the weft yarns include any of silk fibers and rayon fibers.

The woven fabric of paragraph 78, wherein the woven structure includes a warp ends per cm (EPC) of about 60 EPC to about 80 EPC, and a picks per cm (PPC) of about 27 PPC to about 39 PPC.

A woven fabric, comprising:

a plurality of warp yarns, each warp yarn including spun yarns that include either cotton fibers or linen fibers;
a plurality of weft yarns that are interwoven with the plurality of warp yarns to define a woven structure, each weft yarn including spun yarns that include at least linen fibers, wherein the warp and the weft yarns include a count in a range of about 10 Ne to about 80 Ne; and
a composition impregnated into the woven fabric, the composition including a hyaluronic acid component disposed on the woven fabric at a percent add-on of about 0.03 wt.% to about 10 wt.%.

The woven fabric of paragraph 84, wherein the composition further includes at least one of:

an aloe vera extract component disposed on the woven fabric at a percent add-on of about 0.03 wt.% to about 15 wt.%;
a vitamin E component disposed on the woven fabric at a percent add-on of about 0.03 wt.% to about 15 wt.%; and
an antimicrobial component disposed on the woven fabric at a percent add-on of about 0.03 wt.% to about 15 wt.%.

The woven fabric of paragraph 84, wherein either the warp and weft yarns are formed from linen fibers or the warp yarns are formed from cotton fibers and the weft yarns are formed from linen fibers.

The woven fabric of paragraph 84, wherein the woven structure includes an EPC of about 15 EPC to about 23 EPC, and a picks per centimeter (PPC) of about 15 PPC to about 23 PPC.

A knitted fabric, comprising:

a plurality of yarns formed into a knitted fabric that includes courses and wales of loops,
each yarn having include a count in a range of about 10 Ne to about 80 Ne; and
a composition impregnated into the knitted fabric, the composition including a hyaluronic acid component disposed on the knitted fabric at a percent add-on of about 0.03 wt.% to about 10 wt.%.

The knitted fabric of paragraph 88, the composition further including at least one of:

an aloe vera extract component disposed on the knitted fabric at a percent add-on of about 0.03 wt.% to about 15 wt.%;
a vitamin E component disposed on the knitted fabric at a percent add-on of about 0.03 wt.% to about 15 wt.%; and
an antimicrobial component disposed on the knitted fabric at a percent add-on of about 0.03 wt.% to about 15 wt.%.

The knitted fabric of paragraph 88, wherein the plurality of yarns include a blend of cotton and silk fibers.
The knitted fabric of paragraph 90, wherein the yarns include about 50 wt.% to about 80 wt.% cotton fibers and about 20 wt.% to about 70 wt.% silk fibers.
The knitted fabric of paragraph 88, wherein the plurality of yarns include one or more of a) cotton fiber yarns, b) linen fiber yarns, c) rayon fiber yarns, and d) cotton and rayon fiber yarns.
A method of manufacturing a textile article, the method comprising:

weaving a plurality of warp yarns with a plurality of warp yarns to define a woven fabric,
wherein the warp and the weft yarns include a count in a range of about 10 Ne to about 80 Ne;
printing a design onto a face of the woven fabric;
applying a composition to the woven fabric, the composition including a hyaluronic acid component at a concentration of about 0.03 wt.% to about 10 wt.% by weight;
curing the composition onto the woven fabric.

The method of paragraph 93, wherein the composition further includes any one, two or three of the following:

an aloe vera extract component at a concentration of about 0.03 wt.% to about 15 wt.%;
a vitamin E emulsion component at a concentration of about 0.03 wt.% to about 15 wt.%;
an antimicrobial component at a concentration of about 0.03 wt.% to about 15 wt.%; or a catalyst at a concentration of about 0.03 wt.% to about 15 wt.%;

The method of paragraph 93, wherein the applying includes padding the composition onto the woven fabric at a percent wet pick-up (WPU) of about 80 WPU to about 120 WPU.
The method of paragraph 93, wherein the applying includes passing the woven fabric through a bath with a pH of about 5.0 to about 6.0.
The method of paragraph 93, prior to the curing, drying the woven fabric.
The method of paragraph 93, cutting the woven fabric to a shape suitable for a pillow case; and sewing the cut woven fabric into a pillow case.

**Claims**

1. A pillow article configured to provide a skin care benefit, the pillow article comprising:

a pillow case including upper and a lower panels that define an internal space sized to receive a cushion member, the pillow case defining a first end, a second end spaced from the first end along a first direction, and opposed sides spaced apart with respect to each other along a second direction that is perpendicular to the first direction, and at least one of the sides defining a seam that connects the upper panel to the lower panel, the pillow case is configured to be opened to provide access to the inner space, each panel including a plurality of warp yarns and a plurality of weft yarns interwoven with the plurality of warp yarns to define a woven fabric, the woven fabric including a composition disposed thereon that includes a hyaluronic acid component.

2. The pillow article of claim 1, wherein the composition includes at least one of: an aloe vera component, a vitamin E component, and an antimicrobial component.

3. The pillow article of claim 2, wherein the composition includes the hyaluronic acid component, the aloe vera component, the vitamin E component, and the antimicrobial component.

4. The pillow article of claim 3, wherein the hyaluronic acid component is disposed on the fabric at a percent add-on of about 0.03 wt.% to about 10 wt.%.

5. The pillow article of claim 3, wherein the aloe vera component is disposed on the fabric at a percent add-on of about 0.03 wt.% to about 15 wt.%.

6. The pillow article of claim 3, wherein the vitamin E component is disposed on the fabric at a percent add-on of about 0.03 wt.% to about 15 wt.%.

7. The pillow article of claim 3, wherein the antimicrobial component is disposed on the fabric at a percent add-on of about 0.03 wt.% to about 15 wt.%.

8. The pillow article of claim 3, wherein the catalyst is disposed on the fabric at a percent add-on of about 0.03 wt.% to about 15 wt.%.

9. The pillow article of claim 1, wherein the warp yarns are blended yarns that include cotton and silk fibers.

10. The pillow article of claim 9, wherein the blended yarns include about 50 wt.% to about 80 wt.% cotton fibers and about 20 wt.% to about 50 wt.% silk fibers, and preferably wherein the blended yarns include about 70 wt.% cotton fibers and about 30 wt.% silk fibers.

11. The pillow article of claim 1, wherein the weft yarns are blended weft yarns that include cotton and silk fibers.

12. The pillow article of claim 11, wherein the blended yarns include about 50 wt.% to about 80 wt.% cotton fibers and about 20 wt.% to about 50 wt.% silk fibers, and preferably wherein the blended yarns include about 70 wt.% cotton fibers and about 30 wt.% silk fibers.

13. The pillow article of claim 1, wherein the weft yarns are blended weft yarns that include cotton and rayon fibers.

14. The pillow article of claim 13, wherein the blended yarns include about 20 wt.% to about 60 wt.% cotton fibers and about 40 wt.% to about 80 wt.% rayon fibers, and preferably wherein the blended yarns include about 40 wt.% cotton fibers and about 60 wt.% rayon fibers.

15. The pillow article of claim 1, wherein either the warp yarns and the weft yarns are formed from linen fibers or the warp yarns are formed from cotton fibers and the weft yarns are formed from linen fibers.

16. A woven fabric, comprising:

a plurality of warp yarns, each warp yarn including a blend of cotton fibers and silk fibers;
a plurality of weft yarns that are interwoven with the plurality of warp yarns to define a woven structure, each weft yarn including at least cotton fibers, wherein the warp and the weft yarns include a count in a range of about 10 Ne and to about 80 Ne; and
a composition impregnated into the woven fabric, the composition including a hyaluronic acid component disposed on the woven fabric at a percent add-on of about 0.03 wt.% to about 10 wt.%.

17. The woven fabric of claim 16, wherein the composition further includes at least one of:

a. an aloe vera extract component disposed on the knitted fabric at a percent add-on of about 0.03 wt.% to about 15 wt.%;
b. a vitamin E component disposed on the knitted fabric at a percent add-on of about 0.03 wt.% to about 15 wt.%; and
c. antimicrobial component disposed on the knitted fabric at a percent add-on of about 0.03 wt.% to about 15 wt.%.

18. The woven fabric of claim 16, wherein the warp yarns include about 50 wt.% to about 80 wt.% cotton fibers and about 20 wt.% to about 70 wt.% silk fibers, and preferably wherein the warp yarns include about 70 wt.% cotton fibers and about 30 wt.% silk fibers.

19. The woven fabric of claim 16, wherein the weft yarns include silk fibers or rayon fibers.

20. The woven fabric of claim 16, wherein the woven structure includes a warp ends per cm (EPC) of about 60 EPC to about 80 EPC, and a picks per cm (PPC) of about 27 PPC to about 39 PPC.

21. A method of manufacturing a textile article, the method comprising:

weaving a plurality of warp yarns with a plurality of warp yarns to define a woven fabric, wherein the warp and the weft yarns include a count in a range of about 10 Ne to about 80 Ne;
printing a design onto a face of the woven fabric;
applying a composition to the woven fabric, the composition including a hyaluronic acid component at a concentration of about 0.03 wt.% to about 10 wt.% by weight;
curing the composition onto the woven fabric.

22. The method of claim 21, wherein the composition further includes any one, two or three of the following:

a. an aloe vera extract component at a concentration of about 0.03 wt.% to about 15 wt. %;
b. a vitamin E emulsion component at a concentration of about 0.03 wt.% to about 15 wt. %;
c. an antimicrobial component at a concentration of about 0.03 wt.% to about 15 wt. %; or
d. a catalyst at a concentration of about 0.03 wt.% to about 15 wt.%.

23. The method of claim 21, wherein the applying includes padding the composition onto the woven fabric at a percent wet pick-up (WPU) of about 80 WPU to about 120 WPU.

24. The method of claim 21, wherein the applying includes passing the woven fabric through a bath with a pH of about 5.0 to about 6.0.

25. The method of claim 21, prior to the curing, drying the woven fabric.

26. The method of claim 21, cutting the woven fabric to a shape suitable for a pillow case; and sewing the cut woven fabric into a pillow case.

2

10

26

2

12

22

30

16

W

24

6

L

4

28

2

Figure 1

10

12  16  14  20

26

28

18

6

Figure 2

Figure 3

Europäisches
Patentamt

European
Patent Office

Office européen
des brevets

## EUROPEAN SEARCH REPORT

Application Number

EP 16 19 0558

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2014 114519 A (LION CORP) 26 June 2014 (2014-06-26) | 1,8-12, 16,18 | INV. A61K8/73 |
| Y | * paragraphs [0014], [0023], [0034], [0037], [0041], [0045], [0047], [0054]; claim 1 * | 2-7, 13-15, 17,19-26 | D06M15/03 |
| X | JP 2015 144798 A (TANOMURA TAMAKI) 13 August 2015 (2015-08-13) * paragraph [0023]; claims 1,2 * | 1 | |
| Y | WO 2009/129364 A2 (DOW CHEMICAL CO [US]; DAS SUPRIYO [ES]; BILGEN MUSTAFA [US]; REGO JOSE) 22 October 2009 (2009-10-22) * page 1, line 6 - line 8 * * page 16, line 13 - line 26 * * page 23, line 26 - page 24, line 5 * | 21,23-26 | |
| Y | US 2015/245991 A1 (NIHART TROY [US]) 3 September 2015 (2015-09-03) * paragraphs [0039], [0046], [0056], [0061], [0085] * | 2-7,17, 22 | |
| Y | US 6 120 782 A (MANSOURI ZARI [US]) 19 September 2000 (2000-09-19) * column 4, line 21 - line 41 * * column 8, line 61 - column 9, line 7 * * column 12, line 58; claims 1,3 * | 2-7,17, 22 | TECHNICAL FIELDS SEARCHED (IPC) A61K D06M A61Q |
| Y | EP 2 642 021 A1 (OTSUKA PHARMA CO LTD [JP]) 25 September 2013 (2013-09-25) * paragraphs [0018], [0020], [0041], [0042] * | 13-15,19 | |
| Y | WO 86/05413 A1 (EPOC LTD [GB]) 25 September 1986 (1986-09-25) * page 25, line 1 - line 3 * * page 25, line 14 - line 15 * | 20 | |

The present search report has been drawn up for all claims

| Place of search | Date of completion of the search | Examiner |
|---|---|---|
| The Hague | 23 December 2016 | Magrizo, Simeon |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 16 19 0558

This annex lists the patent family members relating to the patent documents cited in the above-mentioned European search report.
The members are as contained in the European Patent Office EDP file on
The European Patent Office is in no way liable for these particulars which are merely given for the purpose of information.

23-12-2016

| Patent document cited in search report | | Publication date | Patent family member(s) | | Publication date |
|---|---|---|---|---|---|
| JP 2014114519 | A | 26-06-2014 | NONE | | |
| JP 2015144798 | A | 13-08-2015 | NONE | | |
| WO 2009129364 | A2 | 22-10-2009 | NONE | | |
| US 2015245991 | A1 | 03-09-2015 | NONE | | |
| US 6120782 | A | 19-09-2000 | AT | 447391 T | 15-11-2009 |
| | | | AU | 724311 B2 | 14-09-2000 |
| | | | AU | 6279996 A | 09-01-1997 |
| | | | CA | 2199993 A1 | 27-12-1996 |
| | | | CN | 1160996 A | 01-10-1997 |
| | | | CN | 1537526 A | 20-10-2004 |
| | | | CN | 1923165 A | 07-03-2007 |
| | | | CN | 101474140 A | 08-07-2009 |
| | | | CN | 102895128 A | 30-01-2013 |
| | | | EP | 0776191 A1 | 04-06-1997 |
| | | | EP | 2145614 A2 | 20-01-2010 |
| | | | NO | 971192 A | 04-04-1997 |
| | | | NZ | 311461 A | 21-12-2001 |
| | | | NZ | 513032 A | 28-03-2003 |
| | | | US | 6096324 A | 01-08-2000 |
| | | | US | 6099849 A | 08-08-2000 |
| | | | US | 6120782 A | 19-09-2000 |
| | | | US | 2001006680 A1 | 05-07-2001 |
| | | | WO | 9641611 A1 | 27-12-1996 |
| EP 2642021 | A1 | 25-09-2013 | EP | 2642021 A1 | 25-09-2013 |
| | | | US | 2013245239 A1 | 19-09-2013 |
| | | | WO | 2012067201 A1 | 24-05-2012 |
| WO 8605413 | A1 | 25-09-1986 | AT | 70985 T | 15-01-1992 |
| | | | AU | 585327 B2 | 15-06-1989 |
| | | | AU | 5546286 A | 13-10-1986 |
| | | | BR | 8605828 A | 04-08-1987 |
| | | | CA | 1279271 C | 22-01-1991 |
| | | | CN | 86102465 A | 31-12-1986 |
| | | | DK | 541786 A | 12-11-1986 |
| | | | EP | 0215087 A1 | 25-03-1987 |
| | | | ES | 296743 U | 01-12-1987 |
| | | | FI | 864590 A | 11-11-1986 |
| | | | GB | 2191107 A | 09-12-1987 |
| | | | IE | 58559 B1 | 06-10-1993 |
| | | | IL | 78134 A | 15-08-1989 |
| | | | JP | 2553060 B2 | 13-11-1996 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 16 19 0558

This annex lists the patent family members relating to the patent documents cited in the above-mentioned European search report.
The members are as contained in the European Patent Office EDP file on
The European Patent Office is in no way liable for these particulars which are merely given for the purpose of information.

23-12-2016

| Patent document cited in search report | Publication date | Patent family member(s) | | Publication date |
|---|---|---|---|---|
| | | JP | S62502314 A | 10-09-1987 |
| | | MX | 169131 B | 23-06-1993 |
| | | NO | 864500 A | 11-12-1986 |
| | | NZ | 215451 A | 31-08-1987 |
| | | OA | 8549 A | 30-09-1988 |
| | | PH | 24558 A | 03-08-1990 |
| | | PT | 82184 A | 01-04-1986 |
| | | US | 4765906 A | 23-08-1988 |
| | | WO | 8605413 A1 | 25-09-1986 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62232323 A

- US 8833075 B